(19) **Europäisches Patentamt / European Patent Office / Office européen des brevets**

(11) **EP 4 700 009 A1**

(12) **EUROPEAN PATENT APPLICATION**
published in accordance with Art. 153(4) EPC

(43) Date of publication:
**25.02.2026 Bulletin 2026/09**

(21) Application number: **24900953.1**

(22) Date of filing: **26.11.2024**

(51) International Patent Classification (IPC):
**C07C 51/377** (2006.01)    **C07C 57/04** (2006.01)

(52) Cooperative Patent Classification (CPC):
**C07C 51/377; C07C 57/04**

(86) International application number:
**PCT/KR2024/018911**

(87) International publication number:
**WO 2025/121766 (12.06.2025 Gazette 2025/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.12.2023 KR 20230176549**

(71) Applicant: **LG Chem, Ltd.**
**Seoul 07336 (KR)**

(72) Inventors:
 • **JUNG, Dabin**
  **Daejeon 34122 (KR)**
 • **SHIN, Daeyoung**
  **Daejeon 34122 (KR)**
 • **KIM, Mi Kyung**
  **Daejeon 34122 (KR)**

 • **JEONG, Hoiin**
  **Daejeon 34122 (KR)**
 • **IM, Yong O**
  **Daejeon 34122 (KR)**
 • **LEE, Jiyoung**
  **Daejeon 34122 (KR)**
 • **KANG, Tae Hun**
  **Daejeon 34122 (KR)**
 • **KIM, Hyunji**
  **Daejeon 34122 (KR)**
 • **KIM, Seungik**
  **Daejeon 34122 (KR)**
 • **LEE, Jongheon**
  **Daejeon 34122 (KR)**

(74) Representative: **Goddar, Heinz J.**
 **Boehmert & Boehmert**
 **Anwaltspartnerschaft mbB**
 **Pettenkoferstrasse 22**
 **80336 München (DE)**

(54) **METHOD FOR PRODUCING ACRYLIC ACID**

(57) The present disclosure relates to a method for producing acrylic acid. Specifically, the present disclosure relates to a method for producing acrylic acid, comprising a method of controlling the reaction temperature so as to maintain the acrylic acid yield in accordance with the progress of the reaction.

[FIG. 1]

EP 4 700 009 A1

**Description**

**[TECHNICAL FIELD]**

CROSS-REFERENCE TO RELATED APPLICATION(S)

This application claims priority to and the benefit of Korean Patent Application No. 10-2023-0176549, filed on December 7, 2023, with the Korean Intellectual Property Office, the disclosure of which is incorporated herein by reference in its entirety.

The present disclosure relates to a method for producing acrylic acid. Specifically, the present disclosure relates to a method for producing acrylic acid, comprising a method of controlling the reaction temperature so as to maintain the acrylic acid yield in accordance with the progress of the reaction.

**[BACKGROUND ART]**

Acrylic acid is an organic compound having both a carboxylic acid and an unsaturated double bond in its molecule, and is used in various industrial fields because it has an extremely simple structure and can be polymerized while being converted into various materials.

Specifically, acrylic acid can be used as polyacrylic acid, tacky-adhesives, paints, etc. required for the production of a superabsorbent polymer, or may be used as a raw material for the production of other types of acrylate-based monomers, or may also be used as a raw material for polymerization with various other monomers such as acrylamide, acrylonitrile, styrene, and alpha olefins.

This acrylic acid is generally produced by using propylene produced in the refining and separation process of crude oil, such as naphtha cracking.

However, recently, as concerns about the depletion of crude oil and environmental issues are increasing, interests in the method for producing acrylic acid using environmentally friendly raw materials are increasing.

Conventionally, in the method of producing acrylic acid through a gas-phase dehydration reaction of lactic acid on an acid catalyst, not only the production of acrylic acid which is a main reaction, but also numerous side reactions occur simultaneously. At this time, coke is formed by the olefin compound generated by the side reaction, which may cover the catalyst active site to cause a catalyst deactivation. Therefore, there is a problem that as the reaction are proceeds, the catalyst deactivation due to coke occurs, so that the acrylic acid yield gradually decreases.

**[DETAILED DESCRIPTION OF THE INVENTION]**

**[Technical Problem]**

It is an object of the present disclosure to provide a method for producing acrylic acid, comprising a method of controlling the reaction temperature so as to maintain the acrylic acid yield at a constant level or higher in accordance with the progress of the reaction.

**[Technical Solution]**

According to the present disclosure, provided is a method for producing acrylic acid, the method comprising the steps of: supplying a feed stream containing lactic acid gas to a dehydration reactor filled with a catalyst to perform a lactic acid dehydration reaction; and controlling the lactic acid dehydration reaction temperature in the form of a positive quadratic function relative to the elapsed time of the dehydration reaction, wherein the lactic acid dehydration reaction is performed in a temperature range of 350°C or more to 400°C or less.

According to one embodiment, the lactic acid dehydration reaction temperature may be maintained at an initial dehydration reaction temperature for 5 hours or more to 35 hours or less after the start of the lactic acid dehydration reaction, and then controlled in the form of a positive quadratic function relative to the elapsed time of the dehydration reaction.

According to one embodiment, the positive quadratic function may have a coefficient range of the highest order term of 0.001 or more to 0.005 or less.

According to one embodiment, the method for producing acrylic acid may satisfy the following Mathematical Formula 1:

[Mathematical Formula 1]

$$T(t_1) < T(t_2); \text{ (when } t_1 < t_2)$$

wherein in Mathematical Formula 1, $t_1$ is a time point at which the first time has elapsed after the start of the lactic acid dehydration reaction, and t2 is a time point at which the second time has elapsed after the start of the lactic acid dehydration reaction,

$T(t_1)$ is the lactic acid dehydration reaction temperature at $t_1$, and $T(t_2)$ is the lactic acid dehydration reaction temperature at $t_2$.

In the method for producing acrylic acid according to one embodiment, the acrylic acid yield is maintained at 45% or more while the lactic acid dehydration reaction proceeds.

According to one embodiment, the lactic acid dehydration reaction may be performed for 20 hours or more to 185 hours or less.

As used herein, the terms "a first," "a second," etc. are used herein to explain various constitutional elements, and these terms are used only to distinguish one constitutional element from another constitutional element.

Further, the technical terms used herein is only to explain exemplary embodiments and is not intended to limit the scope of the present disclosure.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Also, as used herein, in case a layer or an element is mentioned to be formed "on" or "above" layers or elements, it means that the layer or element is directly formed on the layers or elements, or it means that other layers or elements may be additionally formed between the layers, on a subject, or on a substrate.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

When acrylic acid is produced by lactic acid dehydration reaction using a catalyst, coke produced from the by-product of the dehydration reaction covers the active site of the catalyst, and deactivation of the catalyst occurs in accordance with the progress of the reaction. Thus, since the yield of acrylic acid may gradually decrease during the progress of the reaction, a solution to this problem is required.

Therefore, the present disclosure provides a method for producing acrylic acid that can control the dehydration reaction temperature in order to adjust the reaction efficiency according to the degree of catalyst deactivation, and thereby achieve a certain level or higher of acrylic acid yield during the progress of the reaction.

The method for producing acrylic acid according to the present disclosure will be discussed below.

The method for producing acrylic acid according to the present disclosure may comprise a step of supplying a feed stream containing lactic acid gas to a dehydration reactor filled with a catalyst to perform a lactic acid dehydration reaction.

The feed stream containing lactic acid gas supplied to the dehydration reactor may contain vaporized lactic acid gas molecules. As an example, the vaporized lactic acid molecules may be obtained by supplying a stream containing lactic acid to the vaporization reactor and performing a vaporization reaction of lactic acid in the inside of the vaporization reactor.

The catalyst filled in the dehydration reactor is a catalyst for a lactic acid dehydration reaction, and may include at least one selected from the group consisting of a calcium phosphate-based catalyst, a sodium phosphate-based catalyst, and an aluminum phosphate-based catalyst, and the other reaction conditions may be used without particular limitation as long as they are generally used in the technical field to which the present disclosure pertains, unless they are contrary to the contents defined herein.

More specifically, the dehydration catalyst may include $CaSO_4/Na_2SO_4$; $Na_4P_2O_7/CaSO_4$; $Na_4P_2O_7/Ca_3(PO_4)_2$; $NaH_2PO_4$-$NaHCO_3/SiO_2$; $AlPO_4$-$NH_3$; $Ca_3(PO_4)_2/CaSO_4$; $Ca_2P_2O_7$; $Ca_5(PO_4)_3(OH)$, and the like.

In one example, the lactic acid dehydration reaction may be performed in a temperature range of 350°C or more to 400°C or less. That is, the temperature of the lactic acid dehydration reaction may be controlled within a range of 350°C or more to 400°C or less according to the flow of the reaction time.

If the lactic acid dehydration reaction temperature is controlled to be too low, there may be a problem that the lactic acid conversion rate and the acrylic acid yield are significantly reduced. On the contrary, if the temperature is too high, there may be problems that aldehyde may be produced by the carboxyl removal reaction or the carbonyl removal reaction, and the propionic acid production reaction by the reduction of acrylic acid may be promoted, which can increase the content of by-products and denature the dehydration catalyst.

In one example, the method for producing acrylic acid according to the present disclosure may comprise a step of controlling the lactic acid dehydration reaction temperature in the form of a positive quadratic function relative to the elapsed time of the dehydration reaction. Specifically, the lactic acid dehydration reaction temperature may be maintained at the initial dehydration reaction temperature for a prescribed time after the start of the lactic acid dehydration reaction, and then controlled in the form of a positive quadratic function relative to the elapsed time of the dehydration reaction.

Specifically, the lactic acid dehydration reaction temperature may be maintained at the initial dehydration reaction temperature for 5 hours or more to 35 hours or less after the start of the lactic acid dehydration reaction, and then controlled in the form of a positive quadratic function relative to the elapsed time of the dehydration reaction.

More specifically, the lactic acid dehydration reaction temperature may be controlled in the form of a positive quadratic function relative to the elapsed time of the dehydration reaction, after the time period of 5 hours or more, or 10 hours or more, or 20 hours or more to 35 hours or less, 30 hours or less, or 25 hours or less have elapsed since the start of the lactic acid dehydration reaction.

The reason to maintain the initial dehydration reaction temperature for a certain period of time is because the acrylic acid yield and the lactic acid conversion rate are not decreased during the initial certain period of time of the reaction.

The positive quadratic function may have a coefficient range of the highest order term of 0.001 or more to 0.005 or less. Specifically, the coefficient range of the highest order term of the positive quadratic function may be 0.001 or more, or 0.0014 or more to 0.005 or less, or 0.004 or less, or 0.003 or less, or 0.002 or less.

In one example, the method for producing acrylic acid may satisfy the following Mathematical Formula 1:

[Mathematical Formula 1]

$$T(t_1) < T(t_2); \text{ (when } t_1 < t_2)$$

in Mathematical Formula 1, $t_1$ is a time point when the first time has elapsed since the start of the lactic acid dehydration reaction, t2 is the time point when the second time has elapsed since the start of the lactic acid dehydration reaction, $T(t_1)$ is the lactic acid dehydration reaction temperature at $t_1$, and $T(t_2)$ is the lactic acid dehydration reaction temperature at $t_2$.

The Mathematical Formula 1 is intended to show that the dehydration reaction temperature increases with the reaction time.

In one example, the positive quadratic function relative to the reaction time, which is a form in which the dehydration reaction temperature is controlled, can be represented by the following Mathematical Formula 2:

[Mathematical Formula 2]

$$T = 0.0014t^2 - 0.0791t + 367$$

In Mathematical Formula 2, T is the dehydration reaction temperature, and t is the dehydration reaction progress time.

In addition, the temperature at which the dehydration reaction is started may be 350°C or more to 370°C or less, and preferably 360°C or more to 365°C or less.

In one example, the acrylic acid yield may be maintained at 45% or more while the lactic acid dehydration reaction is performed. As the acrylic acid yield is higher, it more economical. Thus, no upper limit thereof has been particularly set, but it may be, for example, 100% or less.

In one example, the lactic acid dehydration reaction may be performed for 20 hours or more to 185 hours or less.

**[ADVANTAGEOUS EFFECTS]**

According to an embodiment of the present disclosure, even if the lactic acid dehydration reaction proceeds for a certain period of time or more, the yield of acrylic acid can be maintained in a certain level or more.

**[BRIEF DESCRIPTION OF THE DRAWINGS]**

FIG. 1 shows the acrylic acid yield relative to the lactic acid dehydration reaction time in the Example and Comparative Example of the present disclosure.
FIG. 2 shows the dehydration reaction temperature control relative to the lactic acid dehydration reaction time in the Example and Comparative Example of the present disclosure.

**[DETAILED DESCRIPTION OF THE EMBODIMENTS]**

Hereinafter, the actions and effects of the invention will be described more specifically with reference to specific examples. However, these examples are presented only as the illustrations of the invention, and the scope of right of the invention is not determined thereby.

**[Preparation of gas-phase lactic acid feed stream]**

A lactic acid aqueous solution with a concentration of 88 wt.% (product name: PURAC H888, available from Corbion) was prepared as a lactic acid raw material. About 1000 g of the lactic acid raw material and 1200 g of distilled water were mixed and refluxed under a temperature condition of about 95°C for about 18 hours to obtain a lactic acid aqueous solution with a concentration of about 40 wt.% in which lactic acid and lactic acid oligomers reached an equilibrium state, which was used as a feed for lactic acid vaporization. A Hastelloy tube with a length of 86 cm and an inner diameter of 2.22 cm was used as a vaporization reactor for the vaporization reaction. The supplied lactic acid feed was configured so as to flow along the inner wall of the vaporization reactor. The internal temperature of the vaporization reactor was set to 300°C. The lactic acid feed prepared above was added at a rate of 0.58 ml/min using a carrier gas so that the lactic acid feed added to the vaporization reactor could flow along the inner wall of the vaporization reactor. Nitrogen was used as the carrier gas, and the carrier gas was supplied at 100 ml/min. The lactic acid molecule feed vaporized in the vaporization reactor was used as the feed for the dehydration reaction.

**[Example 1]**

A dehydration reactor filled with a dehydration catalyst was prepared. The length of the dehydration reactor was 100 cm, the inner diameter was 2.4 cm, and Hastelloy material was used. As the filled catalyst, a composite calcium phosphate-based catalyst consisting of $Ca_2P_2O_7$ and $Ca_5(PO_4)_3(OH)$ formed into cylindrical pellets with a diameter of about 3 mm and a length of about 3 mm was used. At this time, the mixing weight ratio of $Ca_2P_2O_7$ and $Ca_5(PO_4)_3(OH)$ was 7:3.

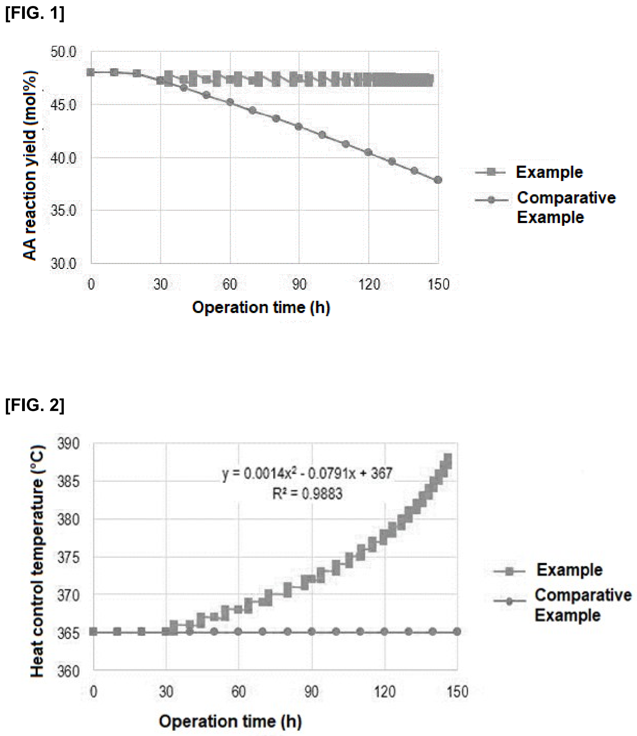

The dehydration reaction starting temperature was set to 365°C, and this temperature was maintained for 33 hours and then gradually increased. At this time, the dehydration reaction temperature was increased to satisfy the following Mathematical Formula 2 (see FIG. 2).

[Mathematical Formula 2]

$$T = 0.0014t^2 - 0.0791t + 367$$

In Mathematical Formula 2, T is the dehydration reaction temperature, and t is the dehydration reaction progress time.

The product that had finished the dehydration reaction in the dehydration reactor was sampled at 40-minute intervals, quantitatively analyzed by HPLC, and the yield of acrylic acid was calculated.

HPLC was performed using high-performance liquid chromatography Agilent 1260 Infinity II, and the obtained sample was diluted 20 times in volume base using distilled water and analyzed. HPLC analysis conditions were as follows.

- Eluent: 0.005 mol H2SO4 (aq)
- Eluent flow rate: 0.4 mL/min
- Column: Aminex HPX-87H
- Column temperature: 10°C
- Detector: UV 210-300 nm
- Analysis time: 70 min
- Analysis pressure: ~70 bar

The acrylic acid yield was calculated by the following Formula.

Acrylic acid yield (%) = Acrylic acid production amount (carbon weight (g)/h) / Lactic acid feed amount (carbon weight (g)/h) * 100

**[Comparative Example 1]**

Acrylic acid was produced in the same manner as in Example 1, except that the dehydration reaction temperature was maintained constant at 365°C.

Similarly, the product that had finished the dehydration reaction in the dehydration reactor was sampled at 40-minute intervals, quantitatively analyzed by HPLC, and the yield of acrylic acid was calculated. The analysis method was the same as in Example 1.

**[Comparison of acrylic acid yield]**

The acrylic acid yields calculated in Example 1 and Comparative Example 1 are shown in the graph in FIG. 1. As a result, it was confirmed that in the case of Example 1, the yield was maintained constant at about 47%, but in the case of Comparative Example, the yield gradually decreased over time. In the case of the Comparative Example, it was understood that this was because the coke produced by the side reaction covered the catalyst active site, and gradually deactivated the catalyst. On the other hand, in the case of Example 1, it was understood that because the reaction rate at which acrylic acid was produced could be increased in response to the catalyst deactivation rate by raising the reaction temperature, the yield was maintained constant.

[Table 1]

| Acrylic acid reaction yield relative to operation time | | | | | | |
|---|---|---|---|---|---|---|
| | 0 hr | 30 hrs | 60 hrs | 90 hrs | 120 hrs | 150 hrs |
| Example 1 | 47% | 47% | 47% | 47% | 47% | 47% |
| Comparative Example 1 | 47% | 46% | 45% | 43% | 41% | 38% |

**Claims**

1.  A method for producing acrylic acid, the method comprising the steps of:

    supplying a feed stream containing lactic acid gas to a dehydration reactor filled with a catalyst to perform a lactic acid dehydration reaction; and
    controlling the lactic acid dehydration reaction temperature in the form of a positive quadratic function relative to the elapsed time of the dehydration reaction,
    wherein the lactic acid dehydration reaction is performed in a temperature range of 350°C or more to 400°C or less.

2.  The method of claim 1,
    wherein the lactic acid dehydration reaction temperature is maintained at an initial dehydration reaction temperature for 5 hours or more to 35 hours or less after the start of the lactic acid dehydration reaction, and then controlled in the form of a positive quadratic function relative to the elapsed time of the dehydration reaction.

3.  The method of claim 1,
    wherein the positive quadratic function has a coefficient range of the highest order term of 0.001 or more to 0.005 or less.

4.  The method of claim 1,

    which satisfies the following Mathematical Formula 1:

[Mathematical Formula 1]

$$T(t_1) < T(t_2); \text{ (when } t_1 < t_2)$$

in Mathematical Formula 1, $t_1$ is a time point at which a first time has elapsed since the start of the lactic acid dehydration reaction, and t2 is a time point at which a second time has elapsed since the start of the lactic acid dehydration reaction, and

$T(t_1)$ is the lactic acid dehydration reaction temperature at $t_1$, and $T(t_2)$ is the lactic acid dehydration reaction temperature at $t_2$.

5. The method of claim 1,
wherein the acrylic acid yield is maintained at 45% or more while the lactic acid dehydration reaction is performed.

6. The method of claim 1,
wherein the lactic acid dehydration reaction is performed for 20 hours or more to 185 hours or less.

EP 4 700 009 A1

[FIG. 1]

[FIG. 2]

$y = 0.0014x^2 - 0.0791x + 367$
$R^2 = 0.9883$

8

## INTERNATIONAL SEARCH REPORT

| International application No. |
|---|
| **PCT/KR2024/018911** |

| A. | CLASSIFICATION OF SUBJECT MATTER |
|---|---|

**C07C 51/377**(2006.01)i; **C07C 57/04**(2006.01)i

According to International Patent Classification (IPC) or to both national classification and IPC

| B. | FIELDS SEARCHED |
|---|---|

Minimum documentation searched (classification system followed by classification symbols)

C07C 51/377(2006.01); B01J 27/053(2006.01); B01J 27/18(2006.01); C07C 51/43(2006.01); C07C 51/44(2006.01); C07C 51/48(2006.01); C07C 51/50(2006.01); C07C 57/065(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아크릴산(acrylic acid), 젖산(lactic acid), 탈수 반응(dehydration reaction), 촉매(catalyst)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2022-0064313 A (LG CHEM, LTD.) 18 May 2022 (2022-05-18)<br>See claims 1 and 4; and paragraph [0066]. | 1-6 |
| A | CN 103638951 A (CHINA WEST NORMAL UNIVERSITY) 19 March 2014 (2014-03-19)<br>See claim 1. | 1-6 |
| A | WO 2022-080905 A1 (LG CHEM, LTD.) 21 April 2022 (2022-04-21)<br>See claims 1 and 10. | 1-6 |
| A | US 2013-0274515 A1 (THE PROCTOR & GAMBLE COMPANY) 17 October 2013 (2013-10-17)<br>See claims 1, 7 and 8. | 1-6 |
| A | KR 10-2016-0122749 A (ARKEMA INC.) 24 October 2016 (2016-10-24)<br>See claim 1. | 1-6 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 March 2025** | **07 March 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

| International application No. |
|---|
| **PCT/KR2024/018911** |

| Patent document cited in search report | | | Publication date (day/month/year) | Patent family member(s) | | | Publication date (day/month/year) |
|---|---|---|---|---|---|---|---|
| KR | 10-2022-0064313 | A | 18 May 2022 | None | | | |
| CN | 103638951 | A | 19 March 2014 | None | | | |
| WO | 2022-080905 | A1 | 21 April 2022 | CN | 116323540 | A | 23 June 2023 |
| | | | | EP | 4206177 | A1 | 05 July 2023 |
| | | | | EP | 4206177 | A4 | 27 March 2024 |
| | | | | JP | 2023-546421 | A | 02 November 2023 |
| | | | | JP | 7618792 | B2 | 21 January 2025 |
| | | | | US | 2023-0382842 | A1 | 30 November 2023 |
| US | 2013-0274515 | A1 | 17 October 2013 | CN | 104245761 | A | 24 December 2014 |
| | | | | CN | 104245761 | B | 22 February 2017 |
| | | | | EP | 2836521 | A1 | 18 February 2015 |
| | | | | EP | 2836521 | B1 | 19 December 2018 |
| | | | | JP | 2015-518481 | A | 02 July 2015 |
| | | | | JP | 6434400 | B2 | 05 December 2018 |
| | | | | US | 2013-0273384 | A1 | 17 October 2013 |
| | | | | US | 2013-0274094 | A1 | 17 October 2013 |
| | | | | US | 2013-0274095 | A1 | 17 October 2013 |
| | | | | US | 2013-0274512 | A1 | 17 October 2013 |
| | | | | US | 2013-0274513 | A1 | 17 October 2013 |
| | | | | US | 2013-0274514 | A1 | 17 October 2013 |
| | | | | US | 2013-0274516 | A1 | 17 October 2013 |
| | | | | US | 2013-0274517 | A1 | 17 October 2013 |
| | | | | US | 2013-0274518 | A1 | 17 October 2013 |
| | | | | US | 2013-0274520 | A1 | 17 October 2013 |
| | | | | US | 2013-0274697 | A1 | 17 October 2013 |
| | | | | US | 8884050 | B2 | 11 November 2014 |
| | | | | US | 9452967 | B2 | 27 September 2016 |
| | | | | WO | 2013-155291 | A1 | 17 October 2013 |
| | | | | WO | 2013-155291 | A4 | 05 December 2013 |
| KR | 10-2016-0122749 | A | 24 October 2016 | CN | 106061934 | A | 26 October 2016 |
| | | | | CN | 106061934 | B | 03 May 2019 |
| | | | | EP | 3110786 | A1 | 04 January 2017 |
| | | | | EP | 3110786 | A4 | 06 September 2017 |
| | | | | EP | 3110786 | B1 | 25 August 2021 |
| | | | | JP | 2017-509613 | A | 06 April 2017 |
| | | | | JP | 6703950 | B2 | 03 June 2020 |
| | | | | KR | 10-2327728 | B1 | 18 November 2021 |
| | | | | US | 10112885 | B2 | 30 October 2018 |
| | | | | US | 2017-0174604 | A1 | 22 June 2017 |
| | | | | WO | 2015-126704 | A1 | 27 August 2015 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230176549